# EUROPEAN PATENT SPECIFICATION

(11) **EP 0 951 887 B1**
(45) Date of publication and mention of the grant of the patent: **27.01.2010**
(21) Application number: 99303060.0
(22) Date of filing: 20.04.1999
(51) Int. Cl.: A61F 13/15

(54) **Absorbent article**
Absorbierender Artikel
Article absorbant

(30) Priority: 20.04.1998 JP 10966198
(43) Date of publication of application: 27.10.1999
(73) Proprietor: UNI-CHARM CORPORATION, Shikokuchuo-shi, Ehime-ken (JP)
(72) Inventor: Mitzutani, Satoshi, c/o R & D. Division, Mitoyo-gun, Kagawa-ken 769-1602 (JP)
(74) Representative: Fitchett, Stuart Paul

(56) References cited:
- EP-A- 0 360 285
- EP-A- 0 419 434
- EP-A- 0 572 033
- WO-A-95/07674
- WO-A-95/17148
- GB-A- 2 296 191
- GB-A- 2 296 437

## Description

The present invention relates to a disposable absorbent article and more particularly to a sanitary napkin adapted to absorb and contain menstrual discharge, a pad adapted to absorb and contain urine for women suffering from incontinence and the like.

In general, conventional sanitary napkins comprise the absorbent laminate which comprises, in turn, a liquid-pervious topsheet, a liquid-impervious backsheet and a liquid-absorbent core disposed between these two sheets. Japanese Patent Application Disclosure Gazette No. Bei2-11138 discloses a sanitary napkin adapted to be convexly deformed towards a wearer's skin by a deformation inducing element having a flexural resistance and underlying the liquidabsorbent core.

According to the disclosure of Japanese Patent Application Disclosure Gazette No. Hei2-11138, the convex deformation occurs merely in accordance with a previously given convex shape of the deformation inducing element. The sanitary napkin of this type will be compressed in a direction of its thickness during operation of packaging to alleviate its bulkiness. If the napkin remains packaged for a long period until it is unwrapped for its actual use, a compressive restoring force of the deformation inducing element may be deteriorated and it may be impossible to obtain a desired convex deformation. Whilst this drawback may be compensated by properly selecting the material for the deformation inducing element, such proper selection is not necessarily easy.

Further prior art arrangements are known from WO 95/17148 and WO 95/07674. WO 95/17148 discloses an elastic member for foreshortening a sanitary napkin in the lateral direction, the elastic member being attached to an absorbent core. WO 95/07674 discloses a sanitary napkin formed with a line of

In view of the problem as has been described above, it is an object of the present invention to provide a sanitary napkin adapted to eliminate the foregoing drawbacks while making the most of advantages provided by the known sanitary napkin.

According to the present invention there is provided a substantially flat and elongate absorbent article as recited in Claim 1.

When the absorbent article according to the present invention is worn by a wearer, the absorbent means is convexly deformed towards the body-facing side and placed closely against the wearer's external genital organs under the lifting effect of the hydrophobic panel member serving as the deformation inducing means.
Fig. 1 is a perspective view of an absorbent article according to the present invention in the form of a sanitary napkin, showing a back side thereof as partially broken away;
Fig. 2 is a plan view showing the back side of the napkin as partially broken away;
Fig. 3 is a schematic view showing the napkin in its cross-section;
Fig. 4 is a view similar to Fig. 3 showing another embodiment of the napkin;
Fig. 5 is a view similar to Fig. 3 showing still another embodiment of the napkin; and
Fig. 6 is a view similar to Fig. 3 showing further another embodiment of the napkin.

Details of an absorbent article according to the present invention will be more fully understood from the description given hereunder based on a sanitary napkin with reference to the accompanying drawings.

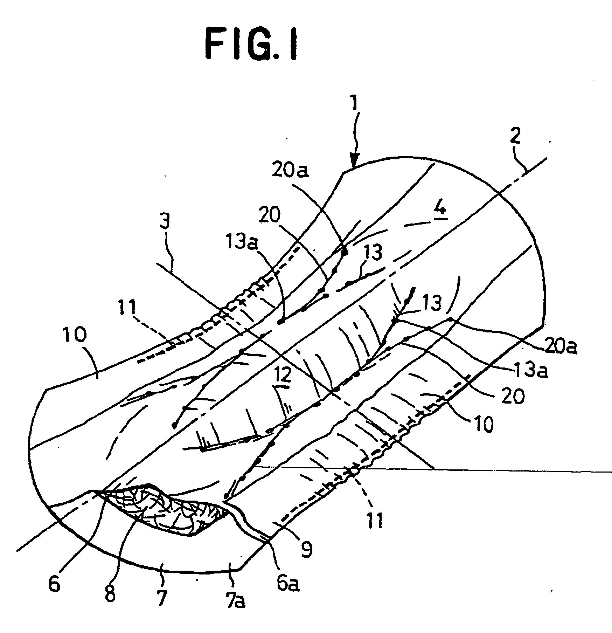

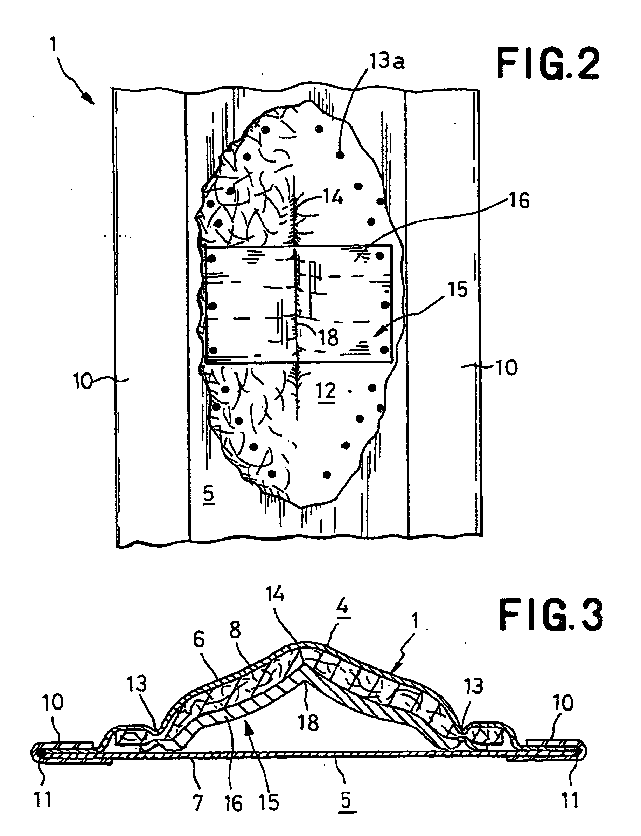

Referring to Figs. 1 - 3, a sanitary napkin comprises absorbent means 1. The absorbent means 1 is geometrically defined by a longitudinal center line 2, a transverse center line 3 being orthogonal to the longitudinal center line 2, a body-facing side 4 and a garment-facing side 5. The absorbent means 1 comprises a liquid-pervious topsheet 6, a liquid-impervious backsheet 7 and a liquid-absorbent core 8 disposed between the topsheet 6 and the backsheet 7. The topsheet 6 and the backsheet 7 may have their sizes larger than the core 8 in length and width and extending outwards beyond a peripheral edge of the core 8. Along each of transversely opposite sides of the napkin, a side flap 10 may be defined by lateral extensions 6a, 7a of the topsheet 6 and the backsheet 7 and longitudinally narrow sheets 9 each folded in two so as to sandwich the lateral extensions 6a, 7a. The side flap 10 may be elasticized by an elastic member 11 provided within its outer edge under appropriate tension in its longitudinal direction and adapted to rise (towards a wearer's skin) as the elastic member 11 contracts.

The topsheet 6, the backsheet 7 and the core 8 may be made of materials well known in the field of sanitary napkin and disposable diaper. The topsheet 6 is made of, for example, a hydrophilic or hydrophobic nonwoven fabric, a plastic film having a plurality of apertures, or a laminate consisting of these materials. The backsheet 7 is made of, a moisture-pervious plastic film or a laminate consisting of such plastic film and hydrophobic nonwoven fabric. The core 8 is made of, for example, a mixture of fluff pulp and superabsorptive polymer particles. As a result, both the topsheet 6 and the backsheet 7 are noticeably flexible while the core 8 is semi-rigid. Though not shown, the topsheet 6 is preferably formed over its entire area with a plurality of liquid guiding apertures each having a diameter larger and therefore a liquid guiding efficiency in a convexly deformable zone 12 as will be described later than in the remaining zone of the topsheet 6. The topsheet 6, if it is of nonwoven-fabric-based, preferably contains thermoplastic fibers at least by an amount required for heat-sealing. The cbre 8 also may contain thermoplastic fibers, if necessary, so far as a desired absorptivity of the core 8 is not affected thereby. The narrow sheet 9 is made of, for example, a hydrophobic nonwoven fabric or a plastic film. The elastic member 11 is made of, for example, natural rubber, synthetic rubber or spandex.

In a region of the absorbent means 1 which is central longitudinally as well as transversely thereof, there are provided a pair of deformation guiding means 13, 13 describing a pair of lines extending longitudinally of the absorbent means 1 and curved outwards symmetrically about a longitudinal center line 2 to define a convexly deformable zone 12 and another pair of deformation guiding means 20 are preferably provided extending also longitudinally of the absorbent means 1 partially in contact with outer sides of the deformation guiding means 13 and curved inwards symmetrically about the center line 2. These deformation guiding means 13, 20 may comprise groups of compressed points (embosses) 13a, 20a or compressed streaks. The deformation guiding means 13, 20 are preferably provided on the body facing side of the absorbent means 1. The absorbent means 1 further includes a single line of deformation guiding means 14 extending on a bottom side (i.e., on the garment-facing side 5) of the core 8 along the longitudinal center line 2 in the convexly deformable zone 12. The deformation guiding means 14 may comprise a single compressed streak or a slit or a group of compressed points (embosses) which divides the core 8 in right and left halves. In the middle of the convexly deformable zone 12, deformation inducing means 15 is preferably secured to a bottom side (i.e., the garment-facing side 5) of the core 8 by means of a hydrophobic adhesive agent such as hot melt adhesive of well known art. The deformation inducing means 15 preferably comprises a rectangular hydrophobic panel member 16. The panel member 16 is substantially deformable to a flat state and preferably has a central zone 18 along the longitudinal center line 2, the central zone 18 normally tending to bend towards the body-facing side 4 (forming so-called latent fold). The central zone 18 of the panel member 16 preferably coincides with the compressed streak 14 serving as the deformation guiding means for the core 8. This central zone 18 preferably comprises a compressed streak having the same shape as the compressed streak 14 and integrally underlying the compressed streak 14. As the napkin is placed against the wearer's external genital organs, the panel member 16 follows a contour of the external genital organs under pressure exerted by the garment and is convexly deformed around the central zone 18 towards the body-facing side 4. In the covexly deformable zone 12, the core 8 is convexly deformed upwards (towards the body-facing side) around the compressed streak 14 serving as the deformation guiding means together with the topsheet 6 under lifting effect due to the deformation of the panel member 16. The deformation causes the core 8 to be spaced from a surface of the backsheet opposed to the core 8 in the convexly deformable zone 12 so that, in the convexly deformable zone 12, the body-facing side 4 may be placed closely against the wearer's external genital organs and leakage of menstrual discharge may be thereby effectively prevented.

Transversely opposite side edges of the panel member 16 preferably extend outwards slightly beyond the deformation guiding means 13, 13 and are preferably bonded to the core 8 in proximity of transversely opposite side edges thereof utilizing the groups of compressed points (emboss-heat-sealing points) 13a. To achieve this bonding, the heat-sealing technique may be replaced by use of hot melt adhesive means.

The panel member 16 may be formed by any one selected from a group consisting of flexible and resilient materials such as a paper sheet, a fibrous sheet, a foamed plastic sheet and a non-foamed plastic sheet or a laminate comprising at least two selected from this group. In general, the panel member 16 has a Gurley stiffness value of 100 - 250 mg, preferably of 130 - 220 mg. The panel member 16 is hydrophobic and free from resilience loss and can induce a desired deformation of the core 8 even when wetted with body fluids such as menstrual discharge. The absorbent means 1, particularly the core 8 thereof generally has a Gurley stiffness value of 50 - 100 mg, preferably of 60 - 90 mg. The core 8 and the panel member 16 integrally bonded together has a Gurley stiffness value of 5 - 80 mg, preferably less than 60 mg when this assembly is deformed around the deformation guiding means 14, 18 towards the bottom surface of the panel member 16.

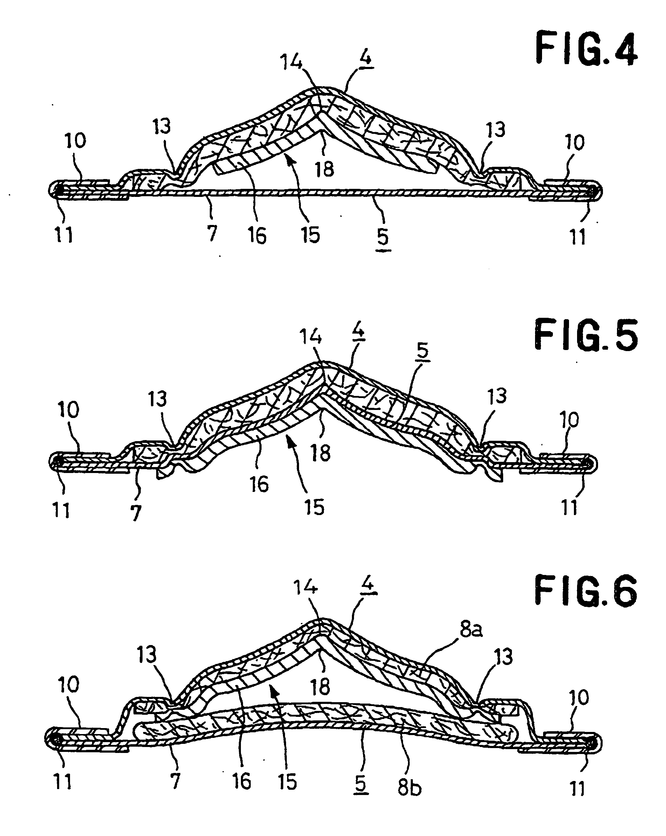

Referring to Fig. 4, the panel member 16 preferably has its transversely opposite side edges lying inside the deformation guiding means 13, respectively, and bonded to the backsheet 7 by means of hot melt adhesive (not shown). Such adhesive bonding may be replaced by the heat-sealing technique.

Referring now to Fig. 5, the panel member 16 is preferably bonded to an outer surface of the backsheet 7 utilizing the groups of compressed points (i.e., emboss-heat-sealing points) 13a. Such heat-sealing technique may be replaced by use of the hot melt adhesive means. The transversely opposite side edges of the panel member 16 may be located inside the deformation guiding means 13, 13, respectively. According to this embodiment also, the absorbent body 1 in the convexly deformable zone 12, i.e., the topsheet 6, the core 8 and the backsheet 7 are integrally and convexly deformed under the effect of the panel member 16.

Referring finally to Fig. 6, the panel member 16 may be disposed between a pair of cores 8a, 8b and bonded integrally to the core 8a. In this embodiment, the panel member 16 is preferably formed with a plurality of apertures (not shown) through which the body fluids can be absorbed by the core 8b.

The absorbent article according to the invention includes the deformation inducing means which is formed separately of the absorbent means. The deformation inducing means is placed on the garment side and has desired hydrophobic nature and rigidity to achieve its function. Specifically, even when wetted with body fluids such as menstrual discharge, the deformation inducing means causes the absorbent means in the convexly deformable zone to be convexly deformed towards the wearer's external genital organs. In this manner, fitting of said absorbent body against the wearer's external genital organs can be improved and leakage of body fluids can be reliably prevented.

The deformation inducing means comprises the hydrophobic panel member which is deformable to a substantially a flat state and causes the absorbent means in the convexly deformable zone to be convexly deformed along the contour of the wearer's external genital organs under the pressure exerted by the garment worn by the wearer. Accordingly, the article can be packaged with the absorbent means and the deformation inducing means being in their flat states.

## Claims

1. A substantially flat and elongate absorbent article having a longitudinal center line (2), a transverse center line (3) being orthogonal to said longitudinal center line (2), a body-facing side (4) and a garment-facing side (5), wherein:
said article includes an absorbent means (1) and deformation inducing means (15) lying adjacent said garment-facing side (5) and causing said absorbent means (1) to be convexly deformed towards the body-facing side (4);
**characterised in that**
said deformation inducing means (15) comprises a hydrophobic panel member (16) having a Gurley stiffness value of at least 100 mg and being substantially deformable to a flat state, and said absorbent means comprises
a first pair of deformation guiding means (13) describing a pair of lines extending longitudinally of said absorbent means (1) and arranged to curve outwardly symmetrically about said longitudinal center line (2) to define a convexly deformable zone (12), and
a line of deformation guiding means (14) on the garment-facing side in said convexly deformable zone extending along said longitudinal center line.

2. The article according to Claim 1, wherein said first deformation guiding means (13) include any of compressed streaks, slits and groups of compressed points and the deformation guiding means (14) comprises a single compressed streak or a slit or a group of compressed points.

3. The article according to Claim 1, wherein said convexly deformable zone (12) of said absorbent means (1) includes second deformation guiding means (20) extending longitudinally of said absorbent means (1) and partially in contact with outer sides of the first deformation guiding means (13) and curved inwardly symmetrically about the center line (2).

4. The article according to Claim 3, wherein said second deformation guiding means (20) include any of compressed points and compressed streaks.

5. The article according to Claim 1, wherein said panel member (16) has resilience.

6. The article according to Claim 1, wherein said absorbent means (1) and said panel member (16) are integrally bonded together along their surfaces opposed to each other and an assembly of the integrated absorbent means (1) and panel member (16) has the maximum Gurley stiffness value of 75 mg when said assembly is deformed around said first deformation guiding means (13) towards a bottom surface of said panel member (16).

7. The article according to Claim 1, wherein said panel member (16) is formed by any one selected from a group consisting of a paper sheet, a fibrous sheet, a foamed plastic sheet and non-foamed plastic sheet of a laminate comprising at least two selected from said group.

8. The article according to Claim 1, wherein said absorbent means (1) comprises a liquid-pervious topsheet (6), a liquid-impervious backsheet (7) and a liquid-absorbent core (8) disposed between said topsheet (6) and said backsheet (7).

9. The article according to Claim 8, wherein, at least in said convexly deformable zone (12), surfaces of said liquid-absorbent core (8) and said backsheet (7) opposed to each other are not integrally bonded together so that these opposed surfaces can be spaced from each other.

10. The article according to Claim 8, wherein said deformation inducing means (15) is disposed between said liquid-absorbent core (8) and said backsheet (7).

11. The article according to Claim 8, wherein said deformation inducing means (15) lies on the outer surface of said backsheet (7).

## Patentansprüche

1. Im Wesentlichen flacher und länglicher absorbierender Artikel mit einer Längsmittellinie (2), einer senkrecht zur besagten Längsmittellinie (2) verlaufenden Quermittellinie (3), einer dem Körper zugewandten Seite (4) und einer der Kleidung zugewandten Seite (5), wobei:
wobei der besagte Artikel ein absorbierendes Mittel (1) und ein Verformung induzierendes Mittel (15) umfasst, das neben der besagten der Kleidung zugewandten Seite (5) angeordnet ist und die konvexe Verformung des besagten absorbierenden Mittels (1) in Richtung der dem Körper zugewandten Seite (4) veranlasst, **dadurch gekennzeichnet, dass**
das besagte Verformung induzierende Mittel (15) ein Wasser abweisendes Stoffbahnelement (16) mit einer Gurley-Steifigkeit von mindestens 100 mg umfasst, das im Wesentlichen zu einem flachen Zustand verformbar ist, und dass das besagte absorbierende Mittel Folgendes umfasst:
ein erstes Paar von Verformungsführungsmitteln (13), die ein Paar von Linien beschreiben, die sich in Längsrichtung des besagten absorbierenden Mittels (1) erstrecken und so angeordnet sind, dass sie zur Definition einer konvex verformbaren Zone (12) symmetrisch um die besagte Längsmittellinie (2) nach außen gekrümmt sind, und
eine sich entlang der besagten Längsmittellinie erstreckende Linie von Verformungsführungsmitteln (14) auf der der Kleidung zugewandten Seite in der besagten konvex verformbaren Zone.

2. Artikel nach Anspruch 1, wobei die besagten ersten Verformungsführungsmittel (13) komprimierte Streifen und/oder Schlitze und/oder Gruppen von komprimierten Punkten umfassen und das Verformungsführungsmittel (14) einen einzelnen komprimierten Streifen, einen Schlitz oder eine Gruppe von komprimierten Punkten umfasst.

3. Artikel nach Anspruch 1, wobei die besagte konvex verformbare Zone (12) des besagten absorbierenden Mittels (1) zweite Verformungsführungsmittel (20) umfasst, die sich in Längsrichtung des besagten absorbierenden Mittels (1) erstrecken, teilweise mit den Außenrändern der besagten ersten Verformungsführungsmittel (13) in Kontakt sind und symmetrisch um die Mittellinie (2) nach innen gekrümmt sind.

4. Artikel nach Anspruch 3, wobei die besagten zweiten Verformungsführungsmittel (20) komprimierte Punkte und/oder komprimierte Streifen umfassen.

5. Artikel nach Anspruch 1, wobei das besagte Stoffbahnelement (16) Elastizität aufweist.

6. Artikel nach Anspruch 1, wobei das besagte absorbierende Mittel (1) und das besagte Stoffbahnelement (16) an ihren einander gegenüber liegenden Oberflächen integriert miteinander verbunden sind und eine aus dem integrierten absorbierenden Mittel (1) und dem Stoffbahnelement (16) bestehende Einheit eine maximale Gurley-Steifigkeit von 75 mg hat, wenn die besagte Einheit um die ersten Verformungsführungsmittel (13) in Richtung der Unterseite des besagten Stoffbahnelements (16) verformt wird.

7. Artikel nach Anspruch 1, wobei das besagte Stoffbahnelement (16) von einem Material gebildet wird, das aus einer aus einem Papierbogen, einem Faserbogen, einer Schaumstofffolie, einer nicht geschäumten Kunststofffolie oder einem aus mindestens zwei aus der besagten Gruppe ausgewählten Stoffen bestehenden Laminat bestehenden Gruppe ausgewählt wird.

8. Artikel nach Anspruch 1, wobei das besagte absorbierende Mittel (1) eine flüssigkeitsdurchlässige obere Lage (6), eine flüssigkeitsundurchlässige rückseitige Lage (7) und einen zwischen der besagten oberen Lage (6) und der besagten rückseitigen Lage (7) angeordneten absorbierenden Kern (8) umfasst.

9. Artikel nach Anspruch 8, wobei mindestens in der besagten konvex verformbaren Zone (12) einander gegenüber liegende Oberflächen des besagten Flüssigkeit absorbierenden Kerns (8) und der besagten rückseitigen Lage (7) nicht integriert miteinander verbunden sind, so dass diese einander gegenüber liegenden Oberflächen voneinander beabstandet sein können.

10. Artikel nach Anspruch 8, wobei das besagte Verformung induzierende Mittel (15) zwischen dem besagten Flüssigkeit absorbierenden Kern (8) und der besagten rückseitigen Lage (7) angeordnet ist.

11. Artikel nach Anspruch 8, wobei das besagte Verformung induzierende Mittel (15) auf der Außenfläche der besagten rückseitigen Lage (7) liegt.

## Revendications

1. Article absorbant sensiblement plat et allongé ayant un axe longitudinal (2), un axe transversal (3) perpendiculaire audit axe longitudinal (2), un côté orienté vers le corps (4) et un côté orienté vers le vêtement (5), dans lequel :
ledit article comprend un moyen absorbant (1) et un moyen entraînant une déformation (15) reposant de manière adjacente par rapport audit côté orienté vers le vêtement (5) et entraînant ledit moyen absorbant (1) à être déformé de manière convexe vers le côté orienté vers le corps (4) ; **caractérisé en ce que**
ledit moyen entraînant une déformation (15) comporte un élément de panneau hydrophobe (16) ayant une valeur de rigidité Gurley d'au moins 100 mg et étant sensiblement déformable en un état plat, et ledit moyen absorbant comporte
une première paire de moyens de guidage de déformation (13) décrivant une paire de lignes s'étendant dans le sens longitudinal par rapport audit moyen absorbant (1) et arrangés pour se courber vers l'extérieur de manière symétrique autour dudit axe longitudinal (2) pour délimiter une zone déformable de manière convexe (12), et
une ligne de moyen de guidage de déformation (14) sur le côté orienté vers le vêtement dans ladite zone déformable de manière convexe s'étendant le long dudit axe longitudinal.

2. Article selon la revendication 1, dans lequel lesdits premiers moyens de guidage de déformation (13) comprennent les uns quelconques parmi des stries comprimées, des fentes ou des groupes de points comprimés et le moyen de guidage de déformation (14) comporte une seule strie comprimée ou une fente ou un groupe de points comprimés.

3. Article selon la revendication 1, dans lequel ladite zone déformable de manière convexe (12) dudit moyen absorbant (1) comprend des deuxièmes moyens de guidage de déformation (20) s'étendant dans le sens longitudinal par rapport audit moyen absorbant (1) et partiellement en contact avec des côtés extérieurs desdits premiers moyens de guidage de déformation (13) et courbés vers l'intérieur de manière symétrique autour de l'axe (2).

4. Article selon la revendication 3, dans lequel lesdits deuxièmes moyens de guidage de déformation (20) comprennent les uns quelconques parmi des points comprimés et des stries comprimées.

5. Article selon la revendication 1, dans lequel ledit élément de panneau (16) a une élasticité.

6. Article selon la revendication 1, dans lequel ledit moyen absorbant (1) et ledit élément de panneau (16) sont intégralement liés ensemble le long de leurs surfaces opposées l'une par rapport à l'autre et un ensemble constitué du moyen absorbant (1) et de l'élément de panneau (16) intégrés a la valeur maximale de rigidité Gurley de 75 mg quand ledit ensemble est déformé autour desdits premiers moyens de guidage de déformation (13) vers une surface inférieure dudit élément de panneau (16).

7. Article selon la revendication 1, dans lequel ledit élément de panneau (16) est formé par l'une quelconque sélectionnée dans un groupe comportant une feuille de papier, une feuille fibreuse, une feuille en matière plastique expansée et une feuille en matière plastique non expansée d'un stratifié comportant au moins deux sélectionnées dans ledit groupe.

8. Article selon la revendication 1, dans lequel ledit moyen absorbant (1) comporte une feuille de dessus perméable aux liquides (6), une feuille de base imperméable aux liquides (7) et une partie centrale absorbant les liquides (8) disposée entre ladite feuille de dessus (6) et ladite feuille de base (7).

9. Article selon la revendication 8, dans lequel, au moins dans ladite zone déformable de manière convexe (12), des surfaces de ladite partie centrale absorbant les liquides (8) et de ladite feuille de base (7) opposées l'une par rapport à l'autre ne sont pas intégralement liées ensemble de telle manière que ces surfaces opposées peuvent être espacées l'une par rapport à l'autre.

10. Article selon la revendication 8, dans lequel ledit moyen entraînant une déformation (15) est disposé entre ladite partie centrale absorbant les liquides (8) et ladite feuille de base (7).

11. Article selon la revendication 8, dans lequel ledit moyen entraînant une déformation (15) repose sur la surface extérieure de ladite feuille de base (7).
